# EUROPEAN PATENT SPECIFICATION

(11) **EP 3 311 792 B1**
(45) Date of publication and mention of the grant of the patent: **23.09.2020**
(21) Application number: 17202262.6
(22) Date of filing: 17.08.2009
(51) Int. Cl.: A61K 8/37, C07C 67/08, C07C 69/533, A61Q 1/04, A61Q 1/10, A61G 5/00, A61Q 5/12, A61Q 17/04, A61Q 19/00, A61Q 19/08, A61Q 19/10

(54) **NATURAL PERSONAL CARE COMPOSITIONS BASED ON UNDECYLENATE ESTER**
NATÜRLICHE KÖRPERPFLEGEZUSAMMENSETZUNGEN VOM UNDECYLENATE ESTER
COMPOSITIONS NATURELLES DE SOINS PERSONNELS À BASE DE L' ESTER UNDÉCYLÉNATE

(30) Priority: 15.08.2008 US 89347 P
(43) Date of publication of application: 25.04.2018
(62) Divisional of application: 09807409.9
(73) Proprietor: Inolex Investment Corporation, Wilmington, DE 19810 (US)
(72) Inventor: Burgo, Rocco, Mullica Hill, NJ New Jersey 08062 (US); Parker, Jeffrey, New Hope, PA Pennsylvania 18938 (US); Winn, Daniel, Kingston, NJ New Jersey 08528 (US)
(74) Representative: Petraz, Gilberto Luigi

(56) References cited:
- US-A- 2 585 053
- US-A- 5 686 087
- US-B1- 6 495 097

## Description

### CROSS REFERENCE TO RELATED APPLICATION

This application claims priority under 35 U.S.C. §119(e) to U.S. Provisional Patent Application No. 61/089,347, filed August 15, 2008.

### BACKGROUND OF THE INVENTION

As the population becomes aware of the potential adverse effects to the body and to the environment associated with use of ingredients derived from fossil fuels, the personal care industry has advanced its search for "natural" ingredients. Although the term "natural" currently has no industry standard definition, efforts are under way by industry trade organizations to devise a more uniform, concise meaning. Currently, it is generally recognized that materials derived from renewable and/or sustainable or otherwise non-fossil fuel sources, are considered to be natural. Personal care compositions containing these materials may be marketed accordingly. Currently, it is the industry consensus that petrochemicals and petrochemically-derived materials are not "natural". There is also a trend within the trade to eliminate animal-derived materials and plant-derived materials that are obtained from the use of genetically modified organisms. Compounds made by certain chemical processes may also be considered unsuitable for use in "natural" personal care compositions.

In skin care, the tactile impression or skinfeel of a product, such as the feeling upon initial application (initial feel), the feeling during spreading of the product over the skin (rub-out), and the feeling after application is complete (after feel), contributes to the product's commercial success or failure. Other characteristics, such as fragrance and taste or lack thereof, may be equally important. Generally, emollients are the components of a composition that are directly related to the "feel" or tactile impression properties, because they provide lubrication, humectancy, and occlusion.

The spreading rate and viscosity are properties of an emollient that contribute to skinfeel or tactile impression. Rapidly spreading/low viscosity products are perceived as "light", whereas slow spreading/higher viscosity products are perceived as "heavy." While heavy feeling products are sometimes preferred, for example, in skin care products such as massage oils, ointments, and barrier creams, lightness as well as a lack of oiliness and/or greasiness is preferred in many applications. Other terms used to describe lightness of a personal care composition may be "dry," "velvety," and "silky." Additionally, in most instances, it is preferable that the emollient(s) is substantially and/or completely free of color and/or taste. It is also desired that they be non-flammable and toxicologically benign.

Vegetable oil emollients have been used in personal care formulations for centuries. Vegetable oils are natural; thus, a personal care formulator preparing a "natural" product may choose a vegetable oil over a petrochemically-derived synthetic, even when the magnitude of benefits realized is lesser as compared to that which would be obtained by use of the petrochemically-derived alternative. Common vegetable oils used in personal care compositions include coconut oil, corn oil, cottonseed oil, canola oil, olive oil, palm oil, peanut oil, safflower oil, sesame oil, soybean oil, sunflower oil and jojoba oil. The viscosities of exemplary vegetable oils are listed in Table 1, and, as can be seen, are relatively high.

**TABLE 1**

| Viscosity of Common Vegetable Oils | |
|---|---|
| Common name | Viscosity, centistokes at 25°C |
| Corn Oil | 65 |
| Canola Oil | 67 |
| Olive Oil | 87 |
| Soybean Oil | 69 |
| Jojoba Oil | 130 |

Low viscosity emollients that can deliver a light feeling to the skin can be made using entirely non-petrochemically-derived starting materials; however, the availability of suitable starting materials is limited. For example, monoesters of low viscosity and light skinfeel can be synthesized by esterifying ethanol from fermentation of corn, sugar cane, beets, and/or other plants with fractionated vegetable fatty acids of lower chain length, normally derived from coconut or palm kernel oil. Although this method can provide low viscosity, the monoesters derived therefrom have a low molecular weight and tend to be volatile, and therefore odorous, which makes their use limited.

Alternatively, "natural" esters for use in personal care compositions may be derived from the esterification of glycerol from vegetable sources with fractionated vegetable derived fatty acids of lower chain length. The most common of these is glyceryl tricaprate/tricaprylate (Lexol GT 8/65, Inolex Chemical Company, Philadelphia, PA) in which the capric and caprylic acid is obtained from splitting and fractionation of coconut or palm kernel oil. The glyceryl tricaprate/tricaprylate material has a viscosity of approximately 25-30 centistokes (at 25°C) and a spreading value of 7.79 (5 minutes, cm²) and has been characterized as having a light skinfeel.

However, the principle remains that odorless, lower viscosity synthetic esters having higher spreading values are considered preferable when one wishes to devise a formulation providing a light skinfeel. For this reason, often petrochemically-derived materials fitting this definition are selected for use over any glyceryl tricaprate/tricaprylate material or other natural materials, since these natural materials do not provide the same performance benefits. Examples of such petrochemically-derived synthetic esters widely used within the personal care industry are neopentyl glycol diheptanoate (LexFeel 7, Inolex Chemical Company, Philadelphia, PA, USA) and isononyl isononanoate (Dermol 99, Alzo International, Sayreville, NJ, USA.) U.S. 4,322,545, 4,323,693, and 4,323,694 to Scala, and U.S. 6,365,629 to Zofchak et. al, also describe wholly or partially petrochemically-derived esters useful in personal care formulations for providing "dry emolliency" and/or "light feeling" to the formulation.

Accordingly, there is a need in the art for esters that can provide a light skinfeel when incorporated as an emollient into personal care formulations and which meet the current "natural" standard. Ideally, such materials would have viscosities and spreading values similar to the petrochemical alternatives. Furthermore, the esters would preferably be low odor, low taste, non-flammable and toxicologically benign.

### BRIEF SUMMARY OF THE INVENTION

The present application discloses a natural ester for topical application to the skin, hair, and/or nails comprising the esterification product of (1) an undecylenic acid derived from a natural source and (2) a vegetable-derived fatty alcohol. The invention includes a personal care product that contains this ester. Also disclosed are methods of preparing a natural personal care composition comprising combining an ester and at least one personal care component, wherein the ester comprises the esterification product of (1) an undecylenic acid derived from a natural source and (2) a vegetable-derived fatty alcohol. Methods of altering the tactile impression and/or skinfeel provided to a user by a personal care composition are part of the present invention.

### DETAILED DESCRIPTION OF THE INVENTION

The present application describes a natural ester for topical application. The present invention includes personal care compositions containing this ester, and methods of altering the skinfeel and/or tactile impression provided to a user by a personal care composition.

The esters disclosed in the invention are natural and can be used to prepare natural personal care compositions. By "natural" it is meant that the starting acids and alcohols are wholly derived from renewable and/or sustainable sources, and are not derived from fossil fuels or any other petrochemical sources.

The invention discloses an ester for topical application to epidermal, mucosal, and/or keratinized surfaces or tissues such as the skin, hair and/or nails. The compositions included herein may be used for topical application to such surfaces/tissues of humans, of animals, and/or for application to animal-derived textiles, furs or skins.

The natural ester includes the esterification product of at least one undecylenic acid that is derived from a natural source. By "natural source", it is meant that the starting acid is derived from a renewable and/or a sustainable resource, and not derived from fossil fuel or any other petrochemical sources. For example, the starting acid may be derived from a vegetable oil, such as those obtained from oil-bearing seeds or other botanical source. Without limitation, exemplary oils include almond oil, castor oil, coconut oil, corn (maize) oil, cottonseed oil, canola oil, flax seed oil, hempseed oil, nut oil(s), olive oil, palm oil, peanut oil, safflower oil, sesame oil, soybean oil, sunflower oil, jojoba oil and combinations of these oils. In an embodiment, preferably the starting acid is derived from castor oil.

The ester disclosed in the present application is prepared by esterifying the acid described above with a vegetable-derived fatty alcohol. The fatty alcohol derives from vegetable-derived fatty alcohols chosen from hexyl alcohol, heptyl alcohol, caprylyl alcohol, nonyl alcohol, decyl alcohol, and undecyl alcohol. Additionally, the ester may be prepared using a mixture or combination of one or more of these vegetable-derived fatty alcohols.

In an embodiment, the ester is prepared from a vegetable-derived fatty alcohol that is heptyl alcohol and the resultant esterification product is heptyl undecylenate.
In an embodiment, the ester is a compound represented by the Formula (I).

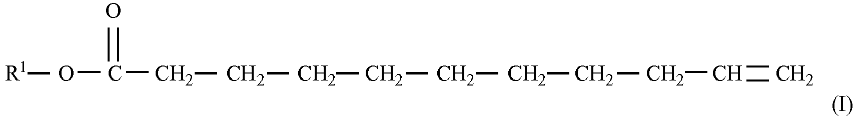

In formula (I), R¹ is an alkyl group. The alkyl group may contain three to twenty-five carbon atoms. However, it may be preferred that the alkyl group of R¹ is a group having six to twelve carbon atoms. Alternatively, it may be preferable that R¹ is chosen from an alkyl group having seven, eight, nine, ten, or eleven carbon atoms. In the formula (I), any of the carbon atoms may be independently substituted or unsubstituted. Suitable substituent groups may include any and all known in the art, such as, for example, methyl groups, butyl groups, ethyl groups, alkoxy groups, branched and/or unbranched alkyl groups, alkyenyl groups, and halogen atoms.

In an embodiment, it may be preferred that R¹ is derived from a vegetable-derived fatty alcohol, such as, for example, hexyl alcohol, heptyl alcohol, caprylyl alcohol, nonyl alcohol, decyl alcohol, and undecyl alcohol.

The selected acid(s) and vegetable-derived fatty alcohol(s) may be esterified by any means known or developed in the art. For example, esterification may be accomplished by application of heat in the presence of a catalyst, or in the absence of a catalyst, using an excess of the more volatile alcohol component to drive the reaction to high conversion. Neutralization, distillation, adsorption, and filtration processes may be used to purify the product. It may be preferred that the method employed is one that leads to an ester of high purity and low color and odor. Such reactions are well within the skill of a person of ordinary skill in the art and are routinely and conventionally executed in the industry.

The ester described herein preferably has a lower viscosity, for example, about 2 to about 10 centistokes at 25°C, alternatively about 3 to about 7 centistokes at 25°C. Preferably, the spreading values of the ester indicate a rapidly spreading material. For example, the spread values may be about 6 to about 8 (5 minute, cm²). These properties contribute to the skinfeel properties/tactile impression of the esters and/or personal care compositions in which it is included; specifically, users perceive a light, silky, and/or dry skinfeel upon application.

The invention includes personal care compositions that include the ester. Personal care compositions may include, for instance oral care products, skin cleansing products, hair cleansing products, nail preparations (nail polish, nail polish removers, cuticle and/or nail treatments), conditioning agents for skin, nails and hair, antiperspirants and deodorants, soaps, hair sprays, gels, hair shampoo, hair conditioner, pomades, powders, cosmetics, compositions that are subsequently impregnated into textiles for cleansing or other purposes, lipstick, lotions for skin and hair, including those containing sunscreens or UV absorbers, bath or shower oils, lip gloss, lipsticks, cosmetics for use in pigmenting the eye area, such as eyebrow pencils or powders, eye shadows, and eye liners, hand lotions and salves, creams and lotions for the facial area, hair creams, mousses, gels, and other styling aids, mascara, foundations for application to the face or tanning products.

There personal care composition contain any additional additives or components useful in formulating a product with the desired end benefits. includes at least one or more vegetable oils in the product, chosen from almond oil, castor oil, coconut oil, corn (maize) oil, cottonseed oil, canola oil, flax seed oil, hempseed oil, nut oil, olive oil, palm oil, peanut oil, safflower oil, sesame oil, soybean oil, sunflower oil, jojoba oil and combinations of these oils.

Surfactants may be included in the personal care composition, such as, for example, an anionic surfactant, a zwitterionic surfactant, a cationic surfactant, a non-ionic surfactant and combinations of these.

Other components are at least one of lipids, alcohols, waxes, pigments, vitamins, fragrances, bleaching agents, antibacterial agents, anti-inflammatory agents, antimycotic agents, thickeners, gums, starches, chitosan, polymeric materials, cellulosic materials, glycerin, proteins, amino acids, keratin fibers, fatty acids, siloxanes, botanical extracts, abrasives and/or exfoliants (chemical or mechanical), anticaking agents, antioxidant agents, binders, biological additives, buffering agents, bulking agents, chelating agents, chemical additives, denaturants, external analgesics, film formers, humectants, opacifying agents, pH adjusters, preservatives, propellants, reducing agents, sunscreen agents, skin darkening agents, essential oils, skin sensates, and combinations of these.

Also disclosed in the present application are methods of preparing a personal care composition including combining the ester of the invention with a personal care composition component. Such component may include any known in the art, such as, for example, those listed above. In an embodiment, the preferred component is a vegetable oil or an essential oil. The invention is also directed to the use of a personal care composition of altering or modifying the tactile impression and/or skinfeel provided to a user by application of an emollient personal care composition of the invention. Such method includes incorporating an ester and at least one component that is chosen from a conventional personal care ingredient component, such as, for example, a gum, a natural oil, a cellulosic compound, a salt, a wax, and/or any of the other components listed above.

All ASTMs referred to below are attached as Exhibit A. EXAMPLE 1 (not part of the invention)

The physical properties of nine esters of the present application are evaluated and compared to two petrochemically-derived esters and one "natural" ester that are currently used in the art.

Kinematic viscosity was tested at 25°C using ASTM (American Society of Testing and Materials, West Conshohocken, Pennsylvania, USA) official method number D-445.

Color was measured using ASTM D-1209, and flash point was determined using ASTM D-92.

Odor was evaluated olfactorily by a human subject.

To test the spreading characteristics, a 110mm Whatman #4 filter paper was positioned horizontally over an open 8 ounce jar. Fifty microliters of each product were then pipetted onto the center of the filter paper. The spreading area of the liquid was then measured at intervals of one, three, and five minutes. More rapidly spreading products will have a higher spreading area at each time interval.

Table 2 shows the properties of the esters of the application in comparison to glyceryl tricaprate/tricaprylate material and two preferred petrochemically-derived emollients.

**TABLE 2**

| SAMPLE | A | B | c | D | E | F | G | H | I |
|---|---|---|---|---|---|---|---|---|---|
| Derivation | Natural | Natural | Natural | Natural | Natural | Natural | Natural | Part Petro. | Fully Petro. |
| Physical Form at R.T. | Liquid | Liquid | Liquid | Liquid | Liquid | Liquid | Liquid | Liquid | Liquid |
| Viscosity at 25°C. cSt | 2.42 | 5.67 | 6.53 | 7.38 | 8.3 | 9.3 | 27.2 | 7.2 | 12.0 |
| Viscosity at 40°C, cSt | 1.73 | 4.0 | 4.57 | 5.05 | 5.7 | 6.2 | 21.1 | 5.6 | N/A |
| Spreading Area (1 minute,) cm² | 7.79 | 7.38 | 7.14 | 6.99 | 6.83 | 6.23 | 4.40 | 6.38 | 7.07 |
| Spreading Area (3 minutes,) cm² | 9.99 | 9.7 | 9.34 | 8.99 | 8.72 | 8.29 | 6.68 | 8.38 | 9.34 |
| Spreading Area (5 minutes,) cm² | 11.44 | 11.24 | 10.55 | 10.36 | 10.08 | 9.25 | 7.79 | 9.89 | 10.26 |
| Odor | Very Mild | Very Mild | Very Mild | Very Mild | Very Mild | Very Mild | Very Mild | Very Mild | Very Mild |
| Color (APHA) | 14 | 5 | 8 | 6 | 6 | 11 | 20 | 17 | 17 |
| Flash Point, °C | 174 | 178 | 180 | 198 | 194 | 180 | 241 | 175 | 175 |

Samples were as follows: A = hexyl undecylenate; B = heptyl undecylenate; C = caprylyl undecylenate; D = nonyl undecylenate, E = decyl undecylenate; F = undecyl undecylenate; G = glyceryl tricaprate/tricaprylate; H = neopentyl glycol diheptanoate; and I = isononyl isononanote.

The results show that the natural esters of the invention possess spreading properties substantially equal to or superior to the petrochemically-derived alternatives.

### EXAMPLE 2

### SPF 15 Skin Lotion

The following example illustrates the use of the invention to prepare a SPF 15 lotion in which the petrochemically-derived neopentyl glycol diheptanoate, an ingredient necessary to provide a light skinfeel in the comparative formulation, is replaced by the natural heptyl undecylenate in the inventive formulation, resulting in a light feeling natural SPF 15 lotion that is entirely free of petrochemically-derived ingredients.

| | INGREDIENT | COMPARATIVE PETROCHEMICAL %, WEIGHT | INVENTIVE NATURAL %, WEIGHT |
|---|---|---|---|
| Phase A | Water | 64.9 | 64.9 |
| | Glycerin | 5.00 | 5.00 |
| | Xanthan Gum | 0.50 | 0.50 |
| | Glucose | 2.00 | 2.00 |
| | Sodium Borate | 0.30 | 0.30 |
| | Sucrose Stearate | 1.50 | 1.50 |
| Phase B | Hempseed Oil | 4.00 | 4.00 |
| | Sucrose Distearate | 1.50 | 1.50 |
| | Nepentyl Glycol Diheptanoate | 7.00 | --- |
| | Heptyl Undecylenate | --- | 7.00 |
| | Beeswax | 5.00 | 5.00 |
| | Aluminum hydroxide | 0.10 | 0.10 |
| | Silica | 0.50 | 0.50 |
| | Titanium Dioxide | 7.00 | 7.00 |
| Phase C | Sandalwood Oil | 0.20 | 0.20 |
| Phase D | Natural Preservative | 0.50 | 0.50 |
| | | 13.3.00 | 100.00 |

The formulation was prepared by combining (in separate vessels) the ingredients of each phase A and phase B and heating each the phase A materials and the phase B materials to 75°C while propeller mixing. Next, the phase A materials and the phase B materials were combined together and homogenized for five minutes. The material was cooled to 45°C while sweep mixing and phase C was added. Cooling was allowed to progress under sweep mixing until the material reached a temperature of 35°C. Phase D was added and mixing continued until the material reached a temperature of 30°C. Mixing was discontinued.

Upon application to the skin, users reported a perception of a light skinfeel.

### EXAMPLE 3

### Natural Tinted Facial Moisturizer

Often, it is desired that creams and lotions that are applied to the face be light in feeling upon initial application, but also provide higher order moisturization benefits that are typically conferred by higher molecular weight, occlusive materials. In this example, the vegetable oils sunflower and coconut are included to provide moisturization, while the ester of the invention, heptyl undecylenate provides perception to the user of an initial light feeling. In the absence of the ester of the invention, this formula is perceived to be heavy, oily and greasy, whereas this formulation is perceived by users to be light because of the "initial feel" contributed by the ester of the invention.

| | INGREDIENT | %, WEIGHT |
|---|---|---|
| Phase A | Water | 67.19 |
| | Glycerin | 5.00 |
| | Xanthan Gum | 0.50 |
| | Glucose | 1.00 |
| | Sodium Borate | 0.30 |
| | Sucrose Stearate | 1.50 |
| Phase B | Sunflower Oil | 4.00 |
| | Coconut Oil | 5.00 |
| | Heptyl Undecylenate | 7.00 |
| | Beeswax | 5.00 |
| | Iron Oxide Black | 0.01 |
| | Iron Oxide Yellow | 0.30 |
| | Iron Oxide Red | 0.70 |
| | Titanium Dioxide | 2.00 |
| Phase C | Natural Preservative | 0.50 |
| | | 100.00 |

The facial moisturizer was prepared using the process of Example 1. Upon application to the facial skin surface, users reported a light skinfeel and sufficient moisturization.

### EXAMPLE 4

### Natural Lipgloss

| INGREDIENT | %, WEIGHT |
|---|---|
| Grapeseed Oil | 29.25 |
| Beeswax | 40.00 |
| Coconut Oil | 10.00 |
| Lanolin | 5.00 |
| Coco butter | 5.00 |
| Tocopheryl Acetate | 0.50 |
| Heptyl Undecylenate | 10.00 |
| Flavor | 0.25 |
| | 100.00 |

The lipgloss was prepared by combining the above ingredients and heating to a temperature of 75°C while propeller mixing until a uniform mixture was obtained. The gloss was cooled to 70°C and packaged.

### EXAMPLE 5

### Natural Bath Oil

| INGREDIENT | %, WEIGHT |
|---|---|
| Sweet Almond Oil | 63.5 |
| Heptyl Undecylenate | 35 |
| Tocopheryl Acetate | 1.0 |
| Fragrance | 0.5 |
| | 100.00 |

The ingredients were combined until uniformly mixed and packaged.

### EXAMPLE 6

### Natural Hair Conditioner

| | INGREDIENT | %, WEIGHT |
|---|---|---|
| Phase A | Water | 74.20 |
| | Glycerin | 5.00 |
| | Honey | 0.50 |
| | Glucose | 2.00 |
| | Betaine | 0.30 |
| | Citric Acid | 0.50 |
| Phase B | Brassicamidopropyl | |
| | | 2.00 |
| | Dimethylamine | |
| | Cetearyl Alcohol | 10.00 |
| | Heptyl Undecylenate | 5.00 |
| Phase C | Preservative | 0.50 |
| | | 100.00 |

The hair conditioner was prepared using the procedure described in Example 1.

### EXAMPLE 7

### Natural Eye Liner/Brow Pencil

| INGREDIENT | %, WEIGHT |
|---|---|
| Sunflower Oil | 36 |
| Heptyl Undecylenate | 25 |
| Japan Wax | 12.00 |
| Carnauba Wax | 10.00 |
| Zinc Oxide | 4.00 |
| Beeswax | 2.00 |
| Tocopheryl Acetate | 1.00 |
| Mica and Titanium dioxide and | |
| Iron Oxide | 10.00 |
| | 100.00 |

The eye liner/brow pencil was prepared by combining the ingredients and heating them to a temperature of 75°C while propeller mixing. Once uniform mixing was achieved, the mixture was cooled to 70°C and packaged.

### EXAMPLE 8

### Natural Eve Shadow

| INGREDIENT | %, WEIGHT |
|---|---|
| Sunflower Oil | 38.00 |
| Heptyl Undecylenate | 25.00 |
| Beeswax | 12.00 |
| Carnauba Wax | 4.00 |
| Zinc Oxide | 8.00 |
| Tocopheryl Acetate | 1.00 |
| Canella wax | 2.00 |
| Mica and Titanium dioxide and Iron Oxide | 10.00 |
| | 100.00 |

The eye shadow was prepared using the procedure described in Example 7 and packaged.

### EXAMPLE 9

### Natural Hand Salve

| INGREDIENT | %, WEIGHT |
|---|---|
| Sweet Almond Oil | 41.80 |
| Heptyl Undecylenate | 32.00 |
| Beeswax | 25.00 |
| Comfrey Root Extract | 1.00 |
| Rosemary Oil | 0.10 |
| Lavender Oil | 0.05 |
| Eucalyptus Oil | 0.05 |
| | 100.00 |

The hand salve was prepared using the procedure described in Example 7 and packaged.

### EXAMPLE 10

### Natural Carrot Day Cream

| | INGREDIENT | %, WEIGHT |
|---|---|---|
| Phase A | Water | 67.60 |
| | Glycerin | 4.00 |
| | Xanthan Gum | 0.40 |
| | Glucose | 1.00 |
| | Sodium Borate | 0.30 |
| Phase B | Wheat germ Oil | 1.50 |
| | Sunflower Oil | 3.00 |
| | Coconut Oil | 4.00 |
| | Heptyl Undecylenate | 5.00 |
| | Beeswax | 5.00 |
| | Tocopheryl Acetate | 0.50 |
| | Sucrose Stearate | 2.00 |
| | Stearic Acid | 4.00 |
| | Orange wax | 1.00 |
| Phase C | Beta-carotene | 0.20 |
| Phase D | Preservative | 0.50 |
| | | 100.00 |

The carrot day cream was prepared using the procedure described in Example 1.

### EXAMPLE 11

| SPF 30 Cream | | |
|---|---|---|
| | INGREDIENT | %, WEIGHT |
| Phase A | Water | 25.50 |
| | Avondale Angustifolia (Lavender) Hydroflorate | 30.00 |
| | Carbomer | 0.20 |
| | Sodium Benzoate | 0.20 |
| | Potassium Sorbate | 0.30 |
| Phase B | Octinoxate | 7.00 |
| | Oxvbenzone | 5.00 |
| | Octisalate | 4.50 |
| | Octocrylene | 5.50 |
| | Titanium Dioxide | 3.50 |
| | Zinc Oxide | 0.50 |
| | Heptyl Undecylenate | 5.00 |
| | Helianthus Annus (Sunflower) Seed Oil | 0.50 |
| | Stearyl Alcohol | 4.00 |
| | Glyceryl Stearate | 1.50 |
| | Steareth 20 | 3.00 |
| | Prunus Amygdalus Dulcis Oil | 0.50 |
| | Camellia Kissi Seed Oil | 0.50 |
| | Steareth 2 | 1.50 |
| Phase C | Camellia Sinensis (Green Tea) Leaf Extract | 0.10 |
| | Aloe Barbadensis (Aloe Vera) Leaf Juice | 0.10 |
| | Deep Sea Algae Extract | 0.10 |
| | Symphytum Officinale (Comfrey) Leaf Extract | 0.10 |
| | Calendula Officinalis (Marigold) Flower Extract | 0.10 |
| | Citrus Grandis (grapefruit) Fruit Extract | 0.50 |
| Phase D | Sodium Hydroxide | 0.10 |
| Phase E | Essential Oil Blend | 0.20 |
| | Total | 100.00 |

This cream was prepared using the procedure of Example 1.

### EXAMPLE 12

| Natural Anti-Age Serum | |
|---|---|
| INGREDIENT | %, WEIGHT |
| Water | 92.60 |
| Corn Starch | 2.00 |
| Lecithin | 1.00 |
| Polysorbate 20 | 1.00 |
| Heptyl Undecylenate | 1.50 |
| Calcium Ascorbate | 0.05 |
| Zinc Ascorbate | 0.05 |
| Ascorbal Phosphate | 0.05 |
| Ascorbic acid | 0.10 |
| Magnesium Ascorbal Palmitate | 0.20 |
| Dextrin Palmitate | 0.15 |
| Camellia Sinensis (Green Tea) Leaf Extract | 0.20 |
| Citrus Grandis (grapefruit) Fruit Extract | 0.20 |
| Fragrance | 0.10 |
| Preservative | 0.80 |
| | 100.00 |

The ingredients for the serum were combined with propeller mixing until uniform, then the serum was packaged.

### EXAMPLE 13

| Hair Texturizing Cream | | |
|---|---|---|
| | INGREDIENT | %, WEIGHT |
| Phase A | Water | 88.67 |
| | Glycerin | 2.00 |
| | Aloe Barbadensis Gel (Aloe Vera | 0.10 |
| | Aminomethyl Propanediol | 0.10 |
| | Carbomer | 0.30 |
| | Benzoic Acid | 0.05 |
| | Phenoxyethanol, | 0.80 |
| | Glycereth 2 Cocoate | 0.20 |
| | Sodium Benzoate | 0.20 |
| Phase B | Stearic Acid | 4.00 |
| | Heptyl Undecylenate | 2.00 |
| | Cetyl Alcohol | 1.00 |
| | Limanthes Alba Seed Oil (Meadowfoam), | 0.50 |
| Phase C | Citrus Medica Limonium Peel Oil (Lemon) | 0.02 |
| | Citrus Aurantum Dulcis Oil (Orange | 0.02 |
| | Citrus Grandis Peel Oil (Grapefruit) | 0.02 |
| | Citrus Aurantium Bergamia Fruit Oil (Bergamot) | 0.02 |
| | | 100.00 |

This cream was prepared using the procedure of Example 1.

### EXAMPLE 14

| Natural Moisturizing Night Cream | | |
|---|---|---|
| | INGREDIENT | %, WEIGHT |
| Phase A | Water | 65.59 |
| | Glycerin | 3.00 |
| | Aspalathus Linearis Leaf Extract (red tea | 0.10 |
| | Copper Gluconate | 0.02 |
| | Zinc Gluconate | 0.02 |
| | Manganese Gluconate | 0.02 |
| | Aloe Barbadensis Leaf Gel | 0.10 |
| | Carbomer | 0.20 |
| | Sodium Benzoate | 0.20 |
| | Sodium PCA | 0.20 |
| | Allantoin | 0.10 |
| | Potassium Cetyl Phosphate | 0.50 |
| | Potassium Sorbate | 0.20 |
| Phase B | Stearic Acid | 3.00 |
| | Heptyl Undecylenate | 10.00 |
| | Stearyl Alcohol | 2.00 |
| | Polysorbate 60 | 1.30 |
| | Simmondsia Chinensis Seed Oil | 2.00 |
| | Prunus Amygdalus Dulcis Oil | 3.00 |
| | Glyceryl Laurate | 1.50 |
| | Cetearyl Alcohol | 3.00 |
| | Ceteareth 20 | 2.00 |
| | Sorbitan Stearate | 1.00 |
| | Tocopheryl Acetate | 0.10 |
| Phase C | Sodium Ascorbyl Phosphate | 0.50 |
| Phase D | Benzyl Alcohol | 0.3 |
| Phase E | Potassium Hydroxide | 0.05 |
| | Total | 100.00 |

This cream was prepared using the procedure of Example 1.

### EXAMPLE 15

### Light Sesame Oil Based Mixture

The following example illustrates how the esters described herein may be admixed with a vegetable oil. Admixtures of this type may be preferred by cosmetic formulators. The following ingredients are combined together in the proportion show below:

| INGREDIENT | %, WEIGHT |
|---|---|
| Sesame Oil | 80.0 |
| Heptyl Undecyleneate | 20.0 |

## Claims

1. A personal care composition comprising a natural ester that is the esterification product of (1) an undecylenic acid derived from a natural source and (2) a vegetable-derived fatty alcohol, wherein the vegetable-derived fatty alcohol is chosen from hexyl alcohol, heptyl alcohol, caprylyl alcohol, nonyl alcohol, decyl alcohol, and undecyl alcohol, and combinations thereof, **characterized in that** it further comprises at least one vegetable oil chosen from almond oil, castor oil, coconut oil, com (maize) oil, cottonseed oil, canola oil, flax seed oil, hempseed oil, nut oil, olive oil, palm oil, peanut oil, safflower oil, sesame oil, soybean oil, sunflower oil, jojoba oil, and combinations thereof, and **in that** it further comprises at least one of a lipid, an alcohol, a wax, a pigment, a vitamin, a fragrance, a bleaching agent, an antibacterial agent, an anti-inflammatory agent, an antimycotic agent, a thickener, a starch, chitosan, a polymeric material, a cellulosic material, glycerin, a protein, an amino acid, a keratin fiber, a fatty acid, a siloxane, an abrasive, an exfoliant, an anticaking agent, an antioxidant agent, a binder, a biological additive, lanolin a buffering agent, a bulking agent, a chelating agent, a chemical additive, a denaturant, an external analgesic, a film former, an humectant, an opacifying agent, a pH adjuster, a preservative, a propellant, a reducing agent, a sunscreen agent, a skin darkening agent, an essential oil, a skin sensate, and combinations of these.

2. The composition of claim 1, wherein the undecylenic acid is derived from castor oil.

3. The composition of claim 1, wherein the vegetable-derived fatty alcohol is heptyl alcohol.

4. The composition of claim 1, wherein the esterification product is heptyl undecylenate.

5. The composition of claim 1, wherein the ester is represented by the Formula (I): 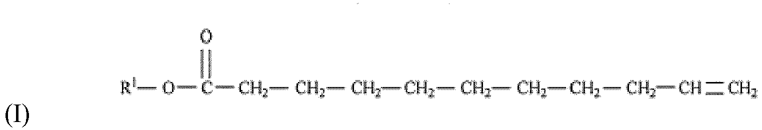 wherein R¹ is chosen from an alkyl group.

6. The composition of claim 5, wherein R¹ is chosen from an alkyl group having six to twelve carbon atoms.

7. The composition of claim 5, wherein R¹ is chosen from an alkyl group having seven, eight, nine, ten, or eleven carbon atoms.

8. The personal care composition of claim 1 further comprising at least one vegetable oil.

9. The composition of claim 1 further comprising at least one component selected from an anionic surfactant, a zwitterionic surfactant, a cationic surfactant, a non-ionic surfactant, and combinations thereof.

10. The composition of claim 1 wherein the composition is natural.

11. Use of a personal care composition for altering the tactile impression and/or skinfeel provided to a user, said personal care composition comprising: an ester and at least one personal care component, wherein the ester comprises the esterification product of (1) undecylenic acid derived from a natural source and a vegetable-derived fatty alcohol, wherein the vegetable-derived fatty alcohol is chosen from hexyl alcohol, heptyl alcohol, caprylyl alcohol, nonyl alcohol, decyl alcohol, and undecyl alcohol, and combinations thereof; and topically applying the personal care composition to the hair, skin, and/or nails of a user, wherein the user perceives a skinfeel or tactile impression of lightness, dryness or silkiness.

12. A personal care composition for use as a suncream, the personal care composition comprising: an ester and at least one personal care component, wherein the ester comprises the esterification product of (1) undecylenic acid derived from a natural source and a vegetable-derived fatty alcohol, wherein the vegetable-derived fatty alcohol is chosen from hexyl alcohol, heptyl alcohol, caprylyl alcohol, nonyl alcohol, decyl alcohol, and undecyl alcohol, and combinations thereof.

## Patentansprüche

1. Körperpflegezusammensetzung, aufweisend einen natürlichen Ester, der das Veresterungsprodukt ist aus (1) einer Undecylensäure, welche aus einer natürlichen Quelle stammt, und (2) einem pflanzlich abgeleiteten Fettalkohol, wobei der pflanzlich abgeleitete Fettalkohol ausgewählt ist aus Hexylalkohol, Heptylalkohol, Caprylylalkohol, Nonylalkohol, Decylalkohol und Undecylalkohol und Kombinationen daraus, **dadurch gekennzeichnet, dass** sie weiterhin wenigstens ein Pflanzenöl aufweist, ausgewählt aus Mandelöl, Rizinusöl, Kokosnussöl, COM (Mais)-Öl, Baumwollsamenöl, Rapsöl, Leinsamenöl, Hanfsamenöl, Nussöl, Olivenöl, Palmöl, Erdnussöl, Safloröl, Sesamöl, Sojabohnenöl, Sonnenblumenöl, Jojobaöl und Kombinationen daraus, und, dass sie weiterhin aufweist wenigstens eines aus einem Lipid, einem Alkohol, einem Wachs, einem Pigment, einem Vitamin, einem Duftstoff, einem Bleichmittel, einem antibakteriellen Mittel, einem entzündungshemmenden Mittel, einem Antimykotikum, einem Verdickungsmittel, einer Stärke, Chitosan, einem Polymermaterial, einem Cellulosematerial, Glycerin, einem Protein, einer Aminosäure, einer Keratinfaser, einer Fettsäure, einem Siloxan, einem Abrasivmittel, einem Peelingmittel, einem Antibackmittel, einem Antioxidationsmittel, einem Bindemittel, einem biologischen Zusatzstoff, Lanolin, einem Puffermittel, einem Füllmittel, einem Chelatbildner, einem chemischen Zusatzstoff, einem Denaturierungsmittel, einem externen Analgetikum, einem Filmbildner, einem Feuchthaltemittel, einem Trübungsmittel, einem pH-regulierenden Mittel, einem Konservierungsmittel, einem Treibmittel, einem Reduktionsmittel, einem Sonnenschutzmittel, einem Hautverdunklungsmittel, einem ätherischen Öl, einem Haut-Sensat und Kombinationen daraus.

2. Zusammensetzung gemäß Anspruch 1, wobei die Undecylensäure von Rizinusöl stammt.

3. Zusammensetzung gemäß Anspruch 1, wobei der pflanzlich abgeleitete Fettalkohol Heptylalkohol ist.

4. Zusammensetzung gemäß Anspruch 1, wobei das Veresterungsprodukt Heptylundecylenat ist.

5. Zusammensetzung gemäß Anspruch 1, wobei der Ester durch die Formel (I) dargestellt ist: 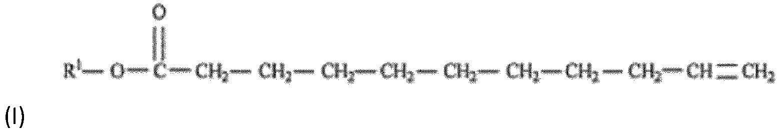 wobei R¹ aus einer Alkylgruppe ausgewählt ist.

6. Zusammensetzung gemäß Anspruch 5, wobei R¹ aus einer Alkylgruppe ausgewählt ist, die sechs bis zwölf Kohlenstoffatome hat.

7. Zusammensetzung gemäß Anspruch 5, wobei R¹ aus einer Alkylgruppe ausgewählt ist, die sieben, acht, neun, zehn oder elf Kohlenstoffatome hat.

8. Körperpflegezusammensetzung gemäß Anspruch 1, weiterhin aufweisend wenigstens ein Pflanzenöl.

9. Zusammensetzung gemäß Anspruch 1, weiterhin aufweisend mindestens eine Komponente ausgewählt aus einem anionischen oberflächenaktiven Stoff, einem zwitterionischen oberflächenaktiven Stoff, einem kationischen oberflächenaktiven Stoff, einen nicht-ionischen oberflächenaktiven Stoff und Kombinationen daraus.

10. Zusammensetzung gemäß Anspruch 1, wobei die Zusammensetzung natürlich ist.

11. Verwendung einer Körperpflegezusammensetzung zum Verändern des einem Verwender bereitgestellten haptischen Eindrucks und/oder Hautgefühls, wobei besagte Körperpflegezusammensetzung aufweist: einen Ester und wenigstens eine Körperpflegekomponente, wobei der Ester das Veresterungsprodukt aus (1) Undecylensäure, welche aus einer natürlichen Quelle stammt, und einem pflanzlich abgeleiteten Fettalkohol aufweist, wobei der pflanzlich abgeleitete Fettalkohol ausgewählt ist aus Hexylalkohol, Heptylakohol, Caprylylalkohol, Nonylalkohol, Decylalkohol und Undecylalkohol und Kombinationen daraus, und zum lokalen Auftragen der Körperpflegezusammensetzung auf die Haare, die Haut und/oder die Nägel eines Verwenders, wobei der Verwender ein Hautgefühl oder einen haptischen Eindruck von Leichtigkeit, Trockenheit oder Seidigkeit wahrnimmt.

12. Körperpflegezusammensetzung zum Verwenden als eine Sonnencreme, wobei die Körperpflegezusammensetzung aufweist: einen Ester und wenigstens eine Körperpflegezusammensetzung, wobei der Ester das Veresterungsprodukt aus (1) Undecylensäure, welche aus einer natürlichen Quelle stammt, und einem pflanzlich abgeleiteten Fettalkohol aufweist, wobei der pflanzlich abgeleitete Fettalkohol ausgewählt ist aus Hexylalkohol, Heptylakohol, Caprylylalkohol, Nonylalkohol, Decylalkohol und Undecylalkohol und Kombinationen daraus.

## Revendications

1. Composition de soins personnels comprenant un ester naturel qui est le produit d'estérification (1) d'un acide undécylénique dérivé d'une source naturelle et (2) d'un alcool gras d'origine végétale, dans laquelle l'alcool gras d'origine végétale est choisi parmi de l'alcool hexylique, alcool heptylique, alcool caprylylique, alcool nonylique, alcool décylique, et alcool undécylique, et des combinaisons de ceux-ci, **caractérisée en ce qu'**elle comprend en outre au moins une huile végétale choisie parmi de l'huile d'amande, huile de ricin, huile de coco, huile de maïs (blé d'Inde), huile de graines de coton, huile de canola, huile de lin, huile de chanvre, huile de noix, huile d'olive, huile de palme, huile d'arachide, huile de carthame, huile de sésame, huile de soja, huile de tournesol, huile de jojoba, et des combinaisons de celles-ci, et **en ce qu'**elle comprend en outre au moins l'un parmi un lipide, un alcool, une cire, un pigment, une vitamine, un parfum, un agent de blanchiment, un agent antibactérien, un agent anti-inflammatoire, un agent antimycotique, un épaississant, un amidon, du chitosane, un matériau polymère, une matière cellulosique, de la glycérine, une protéine, un acide aminé, une fibre de kératine, un acide gras, un siloxane, un abrasif, un exfoliant, un agent anti-agglomérant, un antioxydant, un liant, un additif biologique, de la lanoline, un agent tampon, un agent gonflant, un agent chélatant, un additif chimique, un dénaturant, un analgésique externe, un filmogène, un humectant, un agent opacifiant, un ajusteur de pH, un conservateur, un propulseur, un agent réducteur, un agent d'écran solaire, un agent bronzant, une huile essentielle, un sensibilisant de peau, et des combinaisons de ceux-ci.

2. Composition selon la revendication 1, dans laquelle l'acide undécylénique est dérivé d'huile de ricin.

3. Composition selon la revendication 1, dans laquelle l'alcool gras d'origine végétale est de l'alcool heptylique.

4. Composition selon la revendication 1, dans laquelle le produit d'estérification est de l'undécylénate d'heptyle.

5. Composition selon la revendication 1, dans laquelle l'ester est représenté par la Formule (I) : 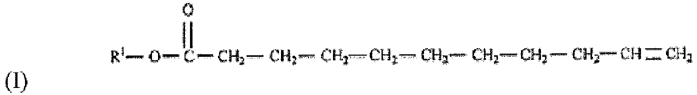 où R¹ est choisi parmi un groupe alkyle.

6. Composition selon la revendication 5, dans laquelle R¹ est choisi parmi un groupe alkyle ayant de six à douze atomes de carbone.

7. Composition selon la revendication 5, dans laquelle R¹ est choisi parmi un groupe alkyle ayant sept, huit, neuf, dix, ou onze atomes de carbone.

8. Composition de soins personnels selon la revendication 1, comprenant en outre au moins une huile végétale.

9. Composition selon la revendication 1, comprenant en outre au moins un composant choisi parmi un tensioactif anionique, un tensioactif zwitterion, un tensioactif cationique, un tensioactif non-ionique, et des combinaisons de ceux-ci.

10. Composition selon la revendication 1, dans laquelle la composition est naturelle.

11. Utilisation d'une composition de soins personnels pour modifier l'impression tactile et/ou la sensation sur peau apportée à un utilisateur, ladite composition de soins personnels comprenant : un ester et au moins un composant de soins personnels, dans laquelle l'ester comprend le produit d'estérification (1) d'un acide undécylénique dérivé d'une source naturelle et d'un alcool gras d'origine végétale, dans laquelle l'alcool gras d'origine végétale est choisi parmi de l'alcool hexylique, alcool heptylique, alcool caprylylique, alcool nonylique, alcool décylique, et alcool undécylique, et des combinaisons de ceux-ci ; et appliquer par voie topique la composition de soins personnels sur les cheveux, la peau et/ou les ongles d'un utilisateur, dans laquelle l'utilisateur perçoit une sensation sur peau ou une impression tactile de légèreté, de dessèchement ou de douceur.

12. Composition de soins personnels à utiliser comme crème solaire, la composition de soins personnels comprenant : un ester et au moins un composant de soins personnels, dans laquelle l'ester comprend le produit d'estérification (1) d'acide undécylénique dérivé d'une source naturelle et d'un alcool gras d'origine végétale, dans laquelle l'alcool gras d'origine végétale est choisi parmi de l'alcool hexylique, alcool heptylique, alcool caprylylique, alcool nonylique, alcool décylique, et alcool undécylique, et des combinaisons de ceux-ci.
